# EUROPEAN PATENT SPECIFICATION

(11) **EP 1 843 157 B1**
(45) Date of publication and mention of the grant of the patent: **16.12.2009**
(21) Application number: 07104460.6
(22) Date of filing: 20.03.2007
(51) Int. Cl.: G01N 33/543

(54) **Method of detecting bio-molecules using field effect transistor without fixing bio-molecules on the gate sensing surface**
Verfahren zur Erkennung von Biomolekülen über einen Feldeffekttransistor ohne Fixierung von Biomolekülen auf der Weichenmessoberfläche
Procédé de détection de biomolécules utilisant un transistor à effet de champ sans fixer les biomolécules sur la surface de détection de porte

(30) Priority: 03.04.2006 KR 20060030170
(43) Date of publication of application: 10.10.2007
(73) Proprietor: Samsung Electronics Co., Ltd., Suwon-si, Gyeonggi-do (KR)
(72) Inventor: Yoo, Kyu-tae, Giheung-gu Yongin-si Gyeonggi-do (KR); Lee, Kyu-sang, Giheung-gu Yongin-si Gyeonggi-do (KR); Chung, Won-seok, Giheung-gu Yongin-si Gyeonggi-do (KR); Shim, Jeo-young, Giheung-gu Yongin-si Gyeonggi-do (KR); Cho, Yeon-ja, Giheung-gu Yongin-si Gyeonggi-do (KR)
(74) Representative: Grünecker, Kinkeldey, Stockmair & Schwanhäusser Anwaltssozietät

(56) References cited:
- WO-A-02/01647
- WO-A-02/086162
- WO-A-2005/043160
- US-A1- 2005 179 065
- SAKURAI T.; HUSIMI Y.: "Real-Time Monitoring of DNA Polymerase Reactions by Micro ISFET pH Sensor" ANALYTICAL CHEMISTRY, vol. 64, 1992, pages 1996-1997,

## Description

### BACKGROUND OF THE INVENTION

### 1. Field of the Invention

The present invention relates to a method of detecting the presence of target bio-molecules or a concentration of the bio-molecules using a field effect transistor.

### 2. Description of the Related Art

A transistor based bio-sensor which includes a transistor is one kind of sensor that detects bio-molecules using electric signals. A semiconductor process is used to manufacture the transistor based bio-sensor, and thus the electric signals can be quickly converted in the transistor based bio-sensor. Accordingly, much research on this kind of sensor has been carried out.

U.S. Patent No. 4,238,757 discloses a field effect transistor (FET) which can be used to detect biological reactions. Using the FET, a bio-sensor measures a current change in an inversion layer of a semiconductor resulting from changes in the surface charge concentration in order to detect an antigen-antibody reaction. Employing a bio-sensor using the FET, a protein among the bio-molecules can be detected. U.S. Patent No. 4,777,019 discloses a sensor for measuring a hybridization of biological monomers with complementary monomers by adsorbing the biological monomers onto the surface of a gate using a FET.

U.S. Patent No. 5,846,708 discloses a method of determining presence of hybridization by an extinction of coupled bio-molecules using a charged couple device (CCD). U.S. Patent Nos. 5,466,348 and 6,203,981 disclose a method of increasing a ratio of signal to noise using a thin film transistor (TFT) with a circuit.

Sakurai T and Husimi Y (ANALYTICAL CHEMISTRY vol. 64 1992, pages 1996-1997) disclose real-time monitoring of DNA polymerase reactions by a micro ISFET pH sensor.

The use of the FET as a bio-sensor decreases costs and reduces the amount of time required to detect the bio-molecules, and the FET is easily used together with an integrated circuit (IC)/MEMS.

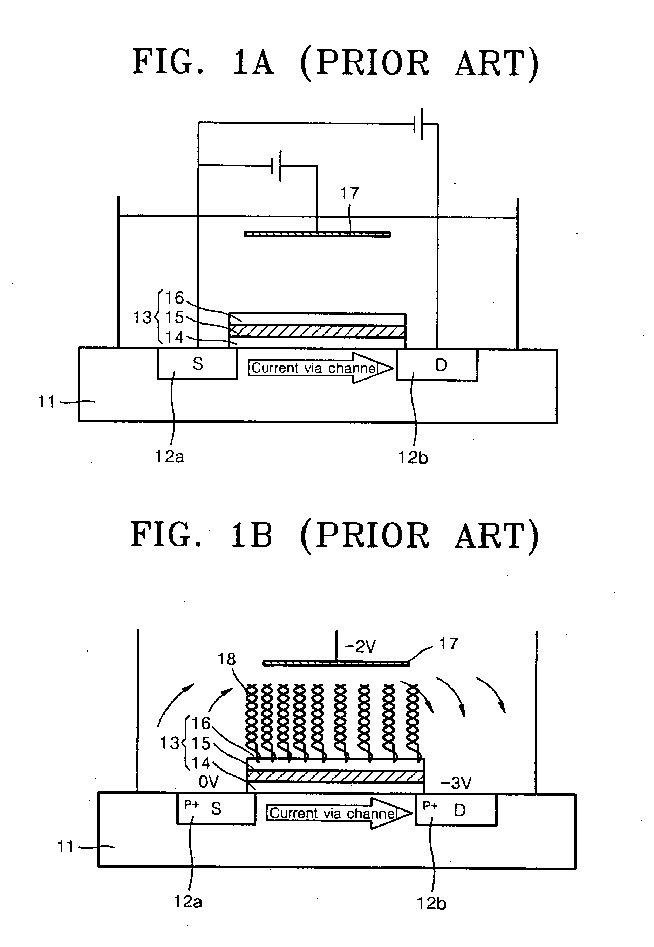

FIG. 1A is a schematic illustration of a structure of a conventional FET sensor. Referring to FIG. 1A, the FET includes a substrate 11 doped with an n-type or a p-type material, a source 12a and a drain 12b which are formed on both sides of the substrate 11 and doped to have the opposite polarity to the substrate 11, and a gate 13 formed on the substrate 11 that contacts the source 12a and the drain 12b. Generally, the gate 13 includes an oxide layer 14, a poly silicon layer 15, and a gate electrode 16. Probe bio-molecules are adhered to the sensing surface of the gate electrode 16 which faces a reference electrode 17. The probe bio-molecule binds to a target bio-molecule through a hydrogen bond, or the like, and the bond is detected using an electrical method.

FIG. 1B is a schematic illustration of a process of immobilizing probe bio-molecules 18 on the surface of a gate electrode 16 of the FET illustrated in FIG. 1A and binding target bio-molecules with the probe bio-molecules 18. Referring to FIG. 1B, a current flowing through a channel varies according to the presence of the immobilized probe bio-molecules 18 on the surface of the gate electrode 16 and the presence of the bond between immobilized probe bio-molecules 18 and the target bio-molecules, and thus the target bio-molecules can be detected.

In all conventional FET structures, bio-molecules such as an oligonucleotide or a PCR product are immobilized on the surface of a gate electrode. An immobilizing technology that is used to manufacture a microarray or a modified technology is used to immobilize the bio-molecules. In International Publication No. WO 03/062811 for example, the surface of a gate is treated with a poly-L-lysine (PLL) having a positive charge using a wet process, DNA is spotted thereon using a spotter and the voltage before and after the spotting is measured.

However, a FET including bio-molecules immobilized on the surface of a gate should be disposed after each use and the response of the sensor is slow. Further, an additional process such as a coating or depositing an additional layer is required to immobilize the bio-molecules and it is expected that differences of characteristics between FETs by the additional process. Also, a spotting cannot easily be used to immobilize the bio-molecules in a lab-on-α-chip.

### SUMMARY OF THE INVENTION

The present invention provides a method of continuously, easily, and accurately detecting the presence of bio-molecules and a concentration of the bio-molecules.

According to an aspect of the present invention, there is provided a method of detecting the presence of bio-molecules or a concentration of the bio-molecules using a field effect transistor without fixing bio-molecules on a sensing surface including: providing a first sample having a first target bio-molecule to a sensing surface of the field effect transistor; and measuring a change in an electric signal of the field effect transistor, wherein the field effect transistor includes: a semiconductor substrate; a source region and a drain region which are formed to be separate on the substrate and doped to have the opposite polarity to the substrate; a channel region disposed between the source region and the drain region; an insulating layer having the sensing surface which is disposed on the channel region and composed of an electrically insulating material; and a reference electrode disposed above and to be separate from the insulating layer.

In an embodiment of the present invention, the method may further include providing a second sample having a second target bio-molecule to the sensing surface of the field effect transistor.

The method may further include washing the sensing surface of the field effect transistor with a solution not having bio-molecules before providing the second sample having the second target bio-molecule to the sensing surface of the field effect transistor.

The electric signal may include at least one of a drain current, a gate-source voltage, and a source-drain voltage.

The bio-molecules may be a nucleic acid or a protein.

The nucleic acid may be one of DNA, RNA, peptide nucleic acid (PNA), locked nucleic acid (LNA), and a hybrid thereof.

The protein may be one of an enzyme, a substrate, an antigen, an antibody, a ligand, an aptamer, and a receptor.

The nucleic acid may be a polymerase chain reaction (PCR) product or a purified PCR product.

The semiconductor substrate may be silicon and the electrically insulating material may be one of a silicon dioxide, a silicon nitride, and a metal oxide.

The substrate may be doped with an n-type material and the source region and the drain region may be doped with a p-type material.

The substrate may also be doped with a p-type material and the source region and the drain region may be doped with an n-type material.

The field effect transistor may be formed in a microchannel.

The substrate may be included in the inner wall of the microchannel.

### BRIEF DESCRIPTION OF THE DRAWINGS

The above and other features and advantages of the present invention will become more apparent by describing in detail exemplary embodiments thereof with reference to the attached drawings in which:
FIG. 1A is a schematic illustration of a structure of a conventional field effect transistor sensor;
FIG. 1B is a schematic illustration of a process of immobilizing probe bio-molecules on the surface of a gate electrode of the field effect transistor illustrated in FIG. 1A and binding target bio-molecules with the probe bio-molecules;
FIG. 2 is a schematic illustration of a structure of a field effect transistor used in a method of detecting bio-molecules of an embodiment of the present invention;
FIG. 3A is a schematic illustration of a procedure of alternately providing PCR products and a washing buffer to the gate electrode of the field effect transistor according to an embodiment of the present invention; and
FIG. 3B is a graph illustrating a change in current when the PCR products and a washing buffer are alternately provided to the gate electrode of the field effect transistor as shown in FIG. 3A.

### DETAILED DESCRIPTION OF THE INVENTION

Hereinafter, the present invention will now be described more fully with reference to the accompanying drawings, in which exemplary embodiments of the invention are shown. The invention may, however, be embodied in many different forms and should not be construed as being limited to the embodiments set forth herein; rather, these embodiments are provided so that this disclosure will be thorough and complete, and will fully convey the concept of the invention to those skilled in the art.

A presence of bio-molecules or a concentration of the bio-molecules can be detected using a field effect transistor according to an embodiment of the present invention without fixing bio-molecules.

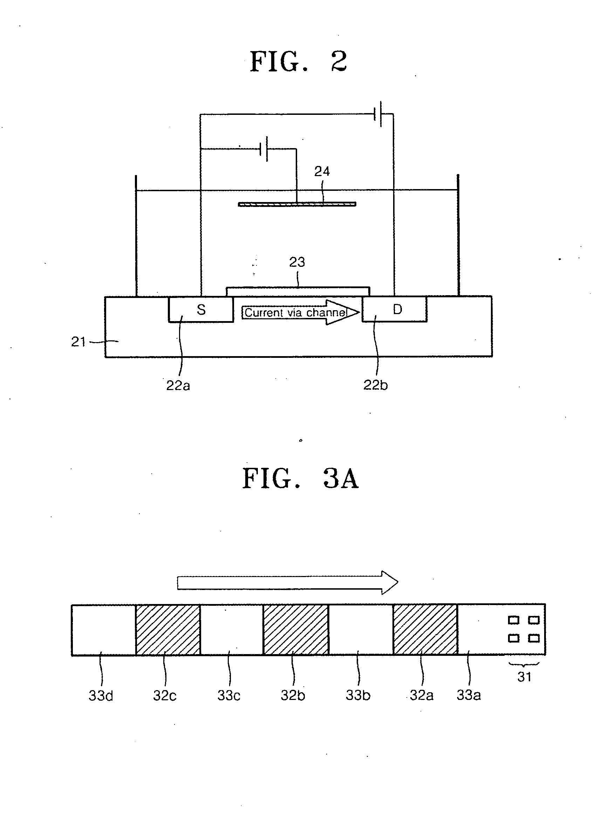

FIG. 2 is a schematic illustration of a structure of a field effect transistor for use in a method of detecting bio-molecules according to an embodiment of the present invention.

Referring to FIG. 2, the field effect transistor for use in the method of detecting bio-molecules includes: a substrate 21 composed of a semiconductor material; a source region 22a and a drain region 22b which are separately formed on the substrate 21 and doped to have the opposite polarity to the substrate 21; a channel region formed between the source region 22a and the drain region 22b; an insulating layer 23 having a sensing surface 23' which is disposed on the channel region and composed of an electrically insulating material; and the reference electrode 24 apart from the insulating layer 23.

The field effect transistor for use in an embodiment of the present invention may be any field effect transistor that is commonly used in a conventional bio-sensor or in a complementary metal oxide semiconductor (CMOS) which can be a n-metal oxide semiconductor (n-MOS) or a p-metal oxide semiconductor (p-MOS). When the substrate 21 is doped with an n-type material, the source 22a and the drain 22b are doped with a p-type material. On the other hand, when the substrate 21 is doped with a p-type material, the source 22a and the drain 22b are doped with an n-type material.

In the field effect transistor, the source 22a may supply carriers such as free electrons or a hole, and the drain 22b may be a region to which the carriers supplied by the source 22a reaches, and the gate electrode 24 may control the flow of the carriers between the source 22a and the drain 22b.

The semiconductor constituting the substrate 21 may be silicon, the electrically insulating material constituting the insulating layer 23 may be any material on which bio-molecules do not become fixed, for example one of a silicon dioxide, a silicon nitride, and a metal oxide. Alternatively, an additional layer composed of another material on which bio-molecules do not become fixed may further be formed on the insulating layer 23.

The field effect transistor may be formed in a microchannel. Here, the substrate 21 may be included in the inner wall of the microchannel, and the gate electrode 24 may be disposed in the microchannel or on the inner wall of the microchannel.

ln the method of detecting bio-molecules according to an embodiment of the present invention, the bio-molecules can be detected using the field effect transistor without fixing bio-molecules.

First, a first sample having a first target bio-molecule is provided to a gate electrode of the field effect transistor.

The bio-molecule may be a nucleic acid or a protein.

The "nucleic acid" is meant to represent various nucleic acids, nucleic acid analogues, and hybrids thereof. For example, the nucleic acid may be one of DNA, RNA, peptide nucleic acid (PNA), locked nucleic acid (LNA), and a hybrid thereof. The nucleic acid may also be an oligonucleotide or a polymerase chain reaction (PCR) product, and preferably a PCR product or a purified PCR product.

The protein may be one of an enzyme, a substrate, an antigen, an antibody, a ligand, an aptamer, and a receptor.

Next, the gate electrode of the field effect transistor may be washed by providing a solution not having bio-molecules into the gate electrode after the first sample is provided to the gate electrode. The solution may be an electrolyte solution.

Then, a second sample having a second target bio-molecule may be provided into the gate electrode of the field effect transistor. The concentration of the second target bio-molecule may be the same as or different from that of the first target bio-molecule.

Changes in electric signal of the field effect transistor are measured during the providing of samples having bio-molecules and a solution not having bio-molecules into the gate electrode.

The electric signal may include at least one of a drain current, a gate-source voltage, and a source-drain voltage.

The method of detecting bio-molecules according to an embodiment of the present invention can be used to detect a corresponding PCR product of a bio-molecule to be detected. A PCR would occur if there are target bio-molecules in the sample, but PCR would not occur if there are not target bio-molecules in the sample. The presence of target bio-molecules and the concentration of the bio-molecules in the sample can be detected by detecting a corresponding PCR product using the method.

In a method of detecting bio-molecules according to an embodiment of the present invention, various bio-molecules can be continuously detected, and also the field effect transistor is not required to be disposed after each use and can be continuously used. The bio-molecules can be quickly detected using the field effect transistor. In addition, since the bio-molecules are not fixed on the field effect transistor, the field effect transistor can be simply manufactured and the distribution of property between FETs that is caused by additional process can be lowered, and thus the bio-molecules can be accurately detected.

Hereinafter, the present invention will be described more specifically with reference to the following Examples. The following Examples are for illustrative purposes and are not intended to limit the scope of the present invention.

### Example 1

### Preparation of a field effect transistor based bio-sensor

A field effect transistor device was fabricated using a XC10-1.0 um CMOS process from X-FAB Semiconductor Foundry service (Germany). The upper surface of a gate was etched to expose silicon oxide, and a gate electrode was formed to be separate from the surface to prepare a field effect transistor as illustrated in FIG. 2.

Then, the surface of the field effect transistor including the exposed silicon oxide and the gate electrode was carefully washed with pure acetone and deionized water and dried. A wet station that is used in a semiconductor manufacturing process was used in washing the substrate. Then, the substrate was dried using a spin dry method.

### Example 2

### Detection of PCR products using the field effect transistor based bio-sensor

It was determined whether a field effect transistor based bio-sensor manufactured in Example 1 could detect a PCR product without fixing the PCR product on the insulating layer, the surface of which senses the PCR product. It was also determined whether the field effect transistor based bio-sensor could detect another PCR product after washing off the previously detected PCR product.

For this, PCR products and a washing solution were alternately provided to the field effect transistor based bio-sensor.

FIG. 3A is a schematic illustration of a procedure of alternately providing PCR products and a washing buffer to the gate electrode of the field effect transistor according to an embodiment of the present invention. Referring to FIG. 3A, a washing solution 33a, a PCR product 32a, a washing solution 33b, a PCR product 32b, a washing solution 33c, a PCR product 32c, and a washing solution 33d were provided to at least one field effect transistor based bio-sensor 31.

0.01 mM phosphate buffer (pH 6.04) was used as the washing solution in Examples.

*Staphylococcus aureus* as a template was amplified through a PCR amplification to obtain the PCR product used in Examples. The base sequence of the forward primer was 5'-(TAG CAT ATC AGA AGG CAC ACC C)-3', and the base sequence of the reverse primer was 5'-(ATC CAC TCA AGA GAG ACA ACA TT)-3'. The amplified PCR product had a size of 240 bp, the pH of the phosphate buffer including the PCR product was 6.47, and the concentration of the PCR product was 10 ng/µℓ.

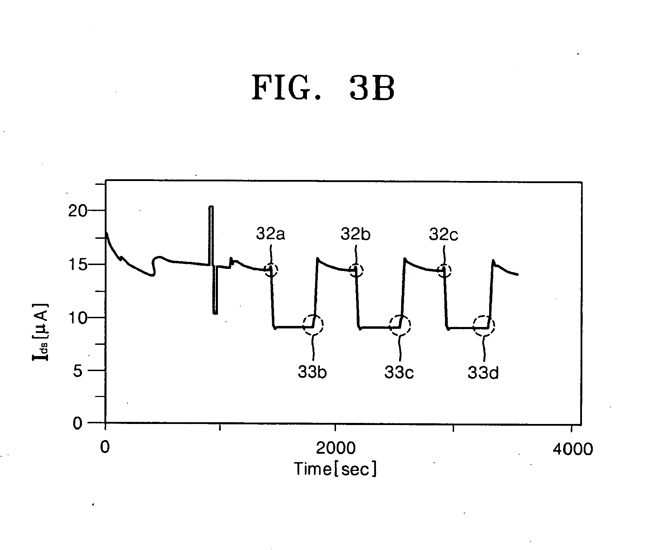

FIG. 3B is a graph illustrating a change in current when the PCR products and a washing buffer are alternately provided to the gate electrode of the field effect transistor as shown in FIG. 3A.

Referring to FIG. 3B, the current rapidly decreased when the PCR products (32a, 32b, and 32c) were provided. On the other hand, the current rapidly increased when the washing solutions (33b, 33c, and 33d) were provided.

The obtained current change was converted into surface voltage change. The results are shown in Table 1.

**Table 1**

| Providing PCR product | | Providing washing solution | |
|---|---|---|---|
| 32a | Decrease by 88.59 mV | 33b | Increase by 90.14 mV |
| 32b | Decrease by 83.34 mV | 33c | Increase by 90.54 mV |
| 32c | Decrease by 78.06 mV | 33d | Increase by 84.83 mV |

As shown in Table 1, when the PCR product was provided, the voltage of the field effect transistor decreased remarkably, and when the washing solution was provided, the voltage of the field effect transistor increased remarkably.

It was measured whether the pH difference between the PCR product and the washing solution influenced the voltage. The pH difference between the PCR product and the washing solution was 0.43, and the voltage change due to the pH difference was merely 10mV. Thus, it is determined that the pH difference does not have a great influence on the voltage.

Accordingly, a plurality of bio-molecules can be continuously, easily, and accurately detected through the method of detecting the presence of bio-molecules or a concentration of the target bio-molecules using a field effect transistor without fixing bio-molecules according to the present invention. The field effect transistor can be semi-permanently used to detect the bio-molecules by washing the field effect transistor.

In the method of detecting the bio-molecules according to the present invention, various bio-molecules can be continuously detected using the field effect transistor, and also the field effect transistor is not required to be disposed after each use and can be continuously used. The bio-molecules can be quickly detected using the field effect transistor. In addition, since the bio-molecules are not fixed on the field effect transistor, the field effect transistor can be simply manufactured and the differences of characteristics between FETs that is caused by additional process can be lowered, and thus the bio-molecules can be accurately detected.

## Claims

1. A method of detecting the presence of bio-molecules or a concentration of the bio-molecules using a field effect transistor without fixing bio-molecules on a sensing surface (23'), the method comprising:
providing a first sample having a first target bio-molecule to a sensing surface (23') of the field effect transistor; and
measuring a change in an electric signal of the field effect transistor,
wherein the field effect transistor comprises: a semiconductor substrate (21); a source region (22a) and a drain region (22b) which are formed to be separate on the substrate (21) and
**characterized in that**
the source region (22a) and the drain region (22b) are doped to have the opposite polarity to the substrate (21);
a channel region is disposed between the source region (22a) and the drain region (22b);
an insulating layer (23) having the sensing surface (23') and composed of an electrically insulating material is disposed on the channel region; and
a reference electrode (24) is disposed above and to be separate from the insulating layer (23).

2. The method of claim 1, further comprising providing a second sample having a second target bio-molecule to the sensing surface (23') of the field effect transistor.

3. The method of claim 2, further comprising washing the sensing surface (23') of the field effect transistor with a solution not having bio-molecules before providing the second sample having the second target bio-molecule to the sensing surface (23') of the field effect transistor.

4. The method of any one of claims 1 to 3, wherein the electric signal is at least one selected from the group consisting of a drain current, a gate-source voltage, and a source-drain voltage.

5. The method of any one of claims 1 to 4, wherein the bio-molecules are a nucleic acid or a protein.

6. The method of claim 5, wherein the nucleic acid is selected from the group consisting of DNA, RNA, peptide nucleic acid (PNA), locked nucleic acid (LNA), and a hybrid thereof.

7. The method of claim 5, wherein the protein is selected from the group consisting of an enzyme, a substrate, an antigen, an antibody, a ligand, an aptamer, and a receptor.

8. The method of claim 5, wherein the nucleic acid is a polymerase chain reaction (PCR) product or a purified PCR product.

9. The method of any one of claims 1 to 8, wherein the semiconductor substrate is silicon and the electrically insulating material is selected from the group consisting of a silicon dioxide, a silicon nitride, and a metal oxide.

10. The method of any one of claims 1 to 9, wherein the field effect transistor is formed in a microchannel.

11. The method of claim 10, wherein the substrate is comprised in the inner wall of the microchannel.

## Patentansprüche

1. Verfahren zum Detektieren der Gegenwart von Bio-Molekülen oder einer Konzentration von Bio-Molekülen mittels eines Feldeffekttransistors, ohne die Bio-Moleküle auf einer Nachweisoberfläche zu fixieren, das Verfahren umfassend: Liefern einer ersten Probe, die ein erstes Ziel-Bio-Molekül hat, an eine Nachweisoberfläche (23') des Feldeffekttransistors; und
Messen einer Änderung in einem elektrischen Signal des Feldeffekttransistors, wobei der Feldeffekttransistor umfasst: ein Halbleitersubstrat (21); einen Source-Bereich (22a) und einen Drain-Bereich (22b), welche auf dem Substrat (21) separat ausgebildet sind;
und
**dadurch gekennzeichnet, dass**
der Source-Bereich (22a) und der Drain-Bereich (22b) dotiert sind, um die entgegengesetzte Polarität zu dem Substrat (21) zu haben;
ein Kanal-Bereich zwischen dem Source-Bereich (22a) und dem Drain-Bereich (22b) angeordnet ist;
eine isolierende Schicht (23), die die Nachweisoberfläche (23') hat und aus einem elektrisch isolierenden Material gebildet ist, auf dem Kanal-Bereich angeordnet ist; und
eine Referenz-Elektrode (24) oberhalb und separat von der isolierenden Schicht (23) angeordnet ist.

2. Verfahren gemäß Anspruch 1, das weiterhin umfasst: das Liefern einer zweiten Probe, die ein zweites Ziel-Bio-Molekül hat, an die Nachweisoberfläche (23') des Feldeffekttransistors.

3. Verfahren gemäß Anspruch 2, das weiterhin umfasst: Waschen der Nachweisoberfläche (23') des Feldeffekttransistors mit einer Lösung, die keine Bio-Moleküle hat, bevor die zweite Probe, die ein zweites Ziel-Bio-Molekül hat, an die Nachweisoberfläche (23') des Feldeffekttransistors gebracht wird.

4. Verfahren gemäß einem der Ansprüche 1 bis 3, wobei als das elektrische Signal mindestens eines aus der Gruppe ausgewählt ist, die einen Drain-Strom, eine Gate-Source-Spannung und eine Source-Drain-Spannung umfasst.

5. Verfahren gemäß einem der Ansprüche 1 bis 4, wobei die Bio-Moleküle eine Nukleinsäure oder ein Protein sind.

6. Verfahren gemäß Anspruch 5, wobei die Nukleinsäure aus der Gruppe ausgewählt ist, die DNA, RNA, Peptid-Nukleinsäure (PNA), verbrückte Nukleinsäure (LNA), und einen Hybrid davon umfasst.

7. Verfahren gemäß Anspruch 5, wobei das Protein aus der Gruppe ausgewählt ist, die ein Enzym, ein Substrat, ein Antigen, ein Antikörper, einen Liganden, ein Aptamer, und einen Rezeptor umfasst.

8. Verfahren gemäß Anspruch 5, wobei die Nukleinsäure ein Polymerase-Kettenreaktionsprodukt (PCR) oder ein gereinigtes PCR Produkt ist.

9. Verfahren gemäß einem der Ansprüche 1 bis 8, wobei das Halbleitersubstrat Silizium ist und das elektrisch isolierende Material aus der Gruppe ausgewählt ist, die ein Siliziumdioxid, ein Siliziumnitrid, und ein Metalloxid umfasst.

10. Verfahren gemäß einem der Ansprüche 1 bis 9, wobei der Feldeffekttransistor in einem Mikrokanal geformt ist.

11. Verfahren gemäß Anspruch 10, wobei das Substrat in der inneren Wand des Mikrokanals enthalten ist.

## Revendications

1. Une méthode pour la détection de la présence de biomolécules ou d'une concentration de biomolécules utilisant un transistor à effet de champ sans fixation de biomolécules sur une surface de détection (23'), le procédé comprenant :
la mise à disposition d'un premier échantillon ayant une première biomolécule cible pour une surface de détection (23) du transistor à effet de champ ; et
mesure d'un changement dans un signal électrique du transistor à effet de champ,
où le transistor à effet de champ comprend : un substrat de semi-conducteur (21) ; une région source (22a) et une région drain (22b) qui sont formés pour être séparés sur le substrat (21) et
**caractérisé par le fait que**
la région source (22a) et la région drain (22b) sont dopées pour avoir la polarité opposée au substrat (21) ;
une région de canal est disposée entre la région source (22a) et la région drain (22b) ;
une couche isolante (23), ayant la surface de détection (23') et composée d'un matériel isolant électrique, est disposée sur la région du canal ; et
une électrode de référence (24) est disposée en haut et de manière séparée de la couche isolante (23).

2. Le procédé selon la revendication 1 , comprenant en outre la mise à disposition d'un second échantillon ayant une seconde biomolécule cible pour la surface de détection (23') du transistor à effet de champ.

3. Le procédé selon la revendication 2, comprenant en outre le lavage la surface de détection (23') du transistor à effet de champ avec une solution ne présentant pas de biomolécules avant de mettre le second échantillon ayant la seconde biomolécule cible à la disposition de la surface de détection (23') du transistor à effet de champ.

4. Le procédé selon l'une des revendications 1 à 3, où le signal électrique est au moins un signal sélectionné dans le groupe comprenant un courant drain, une tension grille-source, et une tension drain source.

5. Le procédé selon l'une des revendications1 à 4, où les biomolécules sont un acide nucléique ou une protéine.

6. Le procédé selon la revendication 5, où l'acide nucléique est sélectionné dans le groupe constitué d'ADN, d'ARN, d'acide nucléique peptidique (PNA), d'acide nucléique verrouillé (LNA), et d'un hybride des mêmes éléments.

7. Le procédé selon la revendication 5, où la protéine est sélectionnée dans le groupe constitué d'une enzyme, d'un substrat, d'un antigène, d'un anticorps, d'un ligand, d'un aptamère, et d'un récepteur.

8. Le procédé selon la revendication 5, où l'acide nucléique est un produit de réaction en chaîne par polymérase (PCR) ou un produit de PCR purifiée.

9. Le procédé selon l'une des revendications 1 à 8, où le substrat de semi-conducteur est en silicium et le matériel d'isolation électrique est sélectionné dans un groupe constitué de dioxyde de silicium, d'azoture de silicium, et d'un oxyde métallique.

10. Le procédé selon l'une des revendications 1 à 9, où le transistor à effet de champ est formé dans un micro canal.

11. Le procédé selon la revendication 10, où le substrat est compris dans la paroi intérieure du micro canal.
